# EUROPEAN PATENT APPLICATION

(11) **EP 3 950 015 A1**
(43) Date of publication of application: **09.02.2022**
(21) Application number: 20779298.7
(22) Date of filing: 13.03.2020
(51) Int. Cl.: A61L 2/18, A61L 9/14

(54) **DECONTAMINATION DEVICE**

(30) Priority: 28.03.2019 JP 2019062333
(71) Applicant: Airex Co., Ltd., Nagoya-shi Aichi 453-0015 (JP)
(72) Inventor: KAWASAKI, Koji, Nagoya-shi Aichi 453-0015 (JP); KAKUDA, Daisuke, Nagoya-shi Aichi 453-0015 (JP); MASUDOME, Jun, Nagoya-shi Aichi 453-0015 (JP); FUTAMURA, Haruka, Nagoya-shi Aichi 453-0015 (JP); YAZAKI, Yukihiro, Nagoya-shi Aichi 453-0015 (JP); KITANO, Tsukasa, Nagoya-shi Aichi 453-0015 (JP); GUO, Zhiqiang, Nagoya-shi Aichi 453-0015 (JP); OGAWA, Ayumi, Nagoya-shi Aichi 453-0015 (JP)
(74) Representative: Lederer & Keller Patentanwälte Partnerschaft mbB
(86) International application number: PCT/JP2020/011183
(87) International publication number: WO 2020/195971

(57) **Abstract**

The present invention provides a decontamination device capable of accomplishing a decontamination effect with a proper amount of decontamination agent supplied to a room to be decontaminated by employing a mist circulation dispersion mechanism and reducing the duration of operations such as aeration to achieve more efficient decontamination works.

The decontamination device of the present invention includes a mist supply means and a mist circulation dispersion mechanism, and the mist supply means converts a chemical for decontamination into a mist for decontamination, and supplies the same to the inside of a working chamber. The mist circulation dispersion mechanism includes vibration boards disposed adjacent to internal wall surfaces of the working chamber, and the vibration boards are subjected to ultrasonic vibration to generate sound flows from board surfaces by an ultrasound in the vertical direction. The mist for decontamination supplied to the inside of the working chamber is pressed by acoustic radiation pressure to circulate and disperse the mist for decontamination in the working chamber.

## Description

### TECHNICAL FIELD

The present invention relates to a decontamination device for decontaminating an inside of a clean room, an isolator device or the like and, more particularly, to a decontamination device including a mist circulation dispersion mechanism.

### BACKGROUND ART

In manufacturing settings for pharmaceutical or food products or in the clinical environment such as operating rooms, the indoor working area must inevitably be kept sterile. Particularly in cases where clean rooms as a working chamber for manufacturing pharmaceutical products are decontaminated, sophisticated decontamination validation needs to be accomplished in accordance with Good Manufacturing Practice (GMP).

In recent years, hydrogen peroxide has widely been used (in the form of a gas or mist) to decontaminate a working chamber such as a clean room (hereinafter referred to as a "room to be decontaminated"). Advantageously, hydrogen peroxide has a strong sterilization effect, and is inexpensively available and effectively utilized as an environmentally-friendly decontamination gas that is ultimately decomposed into oxygen and water.

The following patent document 1 describes that the decontamination effect of hydrogen peroxide is achieved by a condensed film formed by the condensation of a hydrogen peroxide solution on the surface of an object to be decontaminated. Accordingly, in order to accomplish the decontamination effect of the object to be decontaminated, hydrogen peroxide may be supplied in large quantities to make thick or in a higher concentration the resulting condensed film composed of a hydrogen peroxide solution.

### CITATION LIST

### PATENT LITERATURE

Patent Document 1: JP-A-61-004543

### SUMMARY OF INVENTION

### TECHNICAL PROBLEM

In fact, the supply of an excessive amount of hydrogen peroxide to a room to be decontaminated causes extreme condensation, and the resulting condensed film from a high concentration of hydrogen peroxide solution disadvantageously corrodes each manufacturing facility and precision measuring equipment placed inside the room to be contaminated and wall surfaces thereof. After a decontamination work using hydrogen peroxide, aeration is performed with clean air to remove the residual hydrogen peroxide and condensed film inside the room to be decontaminated. However, the supply of such an excessive amount of hydrogen peroxide is problematic due to longer duration required in the aeration operation for removing a condensed film of a high concentration of a hydrogen peroxide solution generated on wall surfaces and other portions of the room to be decontaminated.

Thus, the present invention was made in view of the situation to solve the problems, and has an object to provide a decontamination device capable of accomplishing a decontamination effect with a proper amount of decontamination agent supplied to a room to be decontaminated by employing a mist circulation dispersion mechanism and reducing the duration of operations such as aeration to achieve more efficient decontamination works.

### SOLUTION TO PROBLEM

To solve the aforementioned problem, inventors of the present invention have carried out an extended investigation to find that a condensed film can properly be formed by employing ultrasonic vibration in a mist circulation dispersion mechanism, refining a mist of a hydrogen peroxide solution supplied to a room to be decontaminated, and efficiently circulating the mist in the room to be decontaminated. Based on that technique, the present invention was accomplished.

Specifically, a decontamination device according to the present invention is, according to description in claim 1, a decontamination device (20) placed inside a working chamber (10), the decontamination device including a mist supply means (30) and a mist circulation dispersion mechanism (40), characterized in that
the mist supply means converts a chemical for decontamination into a mist for decontamination, and supplies the same to the inside of the working chamber,
the mist circulation dispersion mechanism includes vibration boards (41, 42) disposed adjacent to internal wall surfaces of the working chamber, and the vibration boards are subjected to ultrasonic vibration to generate sound flows from board surfaces by an ultrasound in the vertical direction, and
the mist for decontamination supplied to the inside of the working chamber is pressed by acoustic radiation pressure to circulate and disperse the mist for decontamination in the working chamber.

Moreover, the present invention is, according to description in claim 2, the decontamination device according to claim 1, characterized in that
the mist circulation dispersion mechanism includes a plurality of vibration boards (241, 242, 243, 244),
the plurality of vibration boards is arranged without board surfaces thereof being opposite each other to generate no stationary wave sound field by an ultrasound generated from each of the vibration boards, and consequently
the mist for decontamination moves so as to rotate inside the working chamber.

Furthermore, the present invention is, according to description in claim 3, the decontamination device according to claim 1 or 2, characterized in that
the vibration board includes a base (45) and a plurality of transmitters (46),
the plurality of transmitters is arranged on a plain surface (45a) of the base so as to be uniform in transmission direction, and the transmitters are operated in the same phase, and
a sound flow is generated by a significantly directional ultrasound from the board surface of the vibration board in the vertical direction by mutually amplifying ultrasounds from the plurality of transmitters in the front direction and mutually canceling out ultrasounds from the plurality of transmitters in the lateral direction.

Moreover, the present invention is, according to description in claim 4, the decontamination device according to any one of claims 1 to 3, characterized in that
the mist for decontamination supplied to the inside of the working chamber is further refined by ultrasonic vibration generated from the vibration board.

Furthermore, the present invention is, according to description in claim 5, the decontamination device according to any one of claims 1 to 4, characterized in that
the decontamination device comprises a control means for changing the frequency and output of the ultrasound generated from the vibration board and/or for transmitting an ultrasound intermittently to control the moving speed of the mist for decontamination circulated and dispersed in the working chamber.

### ADVANTAGEOUS EFFECTS OF INVENTION

According to the above configuration, the decontamination device according to the present invention includes a mist supply means and a mist circulation dispersion mechanism. The mist supply means converts a chemical for decontamination into a mist for decontamination, and supplies the same to the inside of the working chamber. The mist circulation dispersion mechanism includes vibration boards disposed adjacent to internal wall surfaces of the working chamber, and the vibration boards are subjected to ultrasonic vibration to generate sound flows from board surfaces by an ultrasound in the vertical direction. Accordingly, the mist for decontamination supplied to the inside of the working chamber can be refined by ultrasonic vibration and pressed by acoustic radiation pressure to circulate and disperse the mist for decontamination in the working chamber.

Accordingly, the present invention can provide a decontamination device capable of accomplishing a decontamination effect with a proper amount of decontamination agent supplied to a room to be decontaminated by employing a mist circulation dispersion mechanism and reducing the duration of operations such as aeration to achieve more efficient decontamination works.

According to the above configuration, the mist circulation dispersion mechanism may include a plurality of vibration boards. The plurality of vibration boards is arranged without board surfaces thereof being opposite each other to generate no stationary wave sound field by an ultrasound generated from each of the vibration boards. Consequently, the mist for decontamination moves so as to rotate inside the working chamber. Thus, the above operational advantage can more specifically be provided.

According to the above configuration, the vibration board includes a base and a plurality of transmitters, and the plurality of transmitters is arranged on a plain surface of the base so as to be uniform in transmission direction, and the transmitters are operated in the same phase. Consequently, ultrasounds from the plurality of transmitters in the front direction are mutually amplified, and ultrasounds from the plurality of transmitters in the lateral direction are mutually canceled out. Accordingly, a sound flow can be generated by a significantly directional ultrasound from the board surface of the vibration board in the vertical direction. Thus, the above operational advantage can more specifically be provided.

According to the above configuration, the mist for decontamination supplied to the inside of the working chamber is further refined by ultrasonic vibration generated from the vibration board. Accordingly, the above operational advantage can more specifically be provided.

According to the above configuration, the decontamination device may include a control means for changing the frequency and output of the ultrasound generated from the vibration board. The decontamination device may also include a control means for transmitting an ultrasound intermittently. Accordingly, the degree of refinement and the moving speed of the mist for decontamination circulated and dispersed in the working chamber can be controlled. Thus, the above operational advantage can more specifically be provided.

### BRIEF DESCRIPTION OF DRAWINGS

FIG. 1 is a schematic cross-sectional view showing the inside of an isolator including a decontamination device according to a first embodiment viewed from a side;
FIG. 2 is a schematic perspective view showing a plurality of ultrasonic speakers arranged on a speaker base in a vibration board included in the decontamination device in FIG. 1;
FIG. 3 is a schematic cross-sectional view showing the inside of an alternative of the isolator in FIG. 1 viewed from a side;
FIG. 4 is a schematic cross-sectional view showing the inside of a conventional isolator viewed from a side; and
FIG. 5 is a schematic cross-sectional view showing the inside of an isolator including a decontamination device according to a second embodiment viewed from a side.

### DESCRIPTION OF EMBODIMENTS

In the present invention, a "mist" is broadly interpreted as a liquid droplet of a decontamination agent refined and floating in the air, a mixture of a gas and a liquid of the decontamination agent, the decontamination agent between a gas and a droplet in repeated phase-change by condensation and evaporation, and the like. In terms of particle size as well, the mist is also broadly interpreted to include mists, fogs, and liquid droplets, which can be subclassified.

Accordingly, the mist according to the present invention is categorized into a "mist" (the size may be defined as 10 µm or less) or a "fog" (the size may be defined as 5 µm or less), and a mist having a larger particle size. In the present invention, ultrasonic vibration converts even a mist, a fog and a liquid droplet sized 3 to 10 µm into equalized ultrafine particles 3 µm or less to provide sophisticated decontamination effects (later described).

The decontamination according to the present invention will be described with reference to each embodiment. The present invention is not restricted to each of the following embodiments.

### <First embodiment>

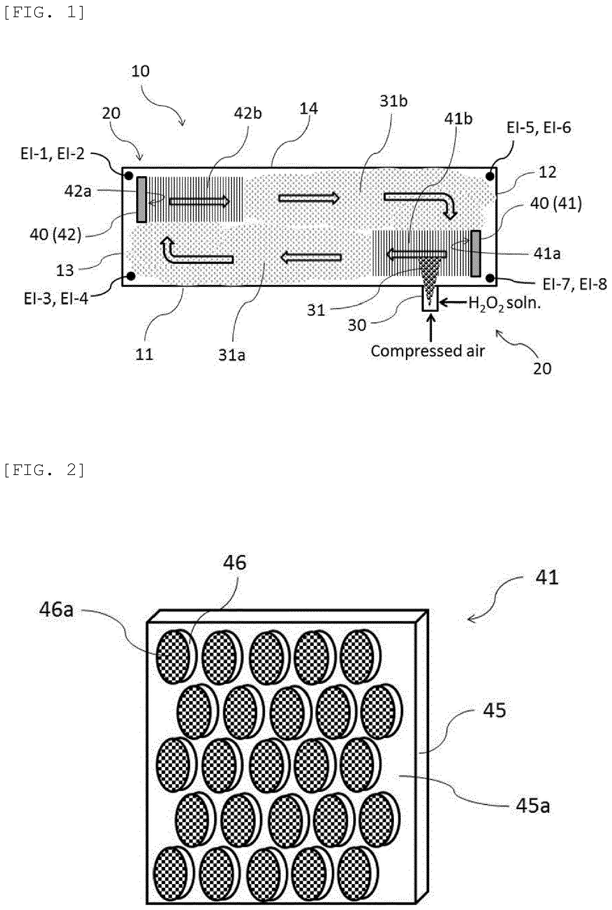

A first embodiment will be described by illustrating an isolator as a work room to be decontaminated. FIG. 1 is a schematic cross-sectional view showing the inside of an isolator including a decontamination device according to the first embodiment viewed from a side.

In FIG. 1, an isolator 10 includes a decontamination device 20 inside thereof. The decontamination device 20 is composed of a mist supply unit 30, a mist circulation dispersion unit 40, and a control unit (not shown) . In this first embodiment, the mist supply unit 30 used is a two-fluid spray nozzle 30 placed on a bottom wall surface 11 of the isolator 10. In this first embodiment, the decontamination agent used is a hydrogen peroxide solution (H₂O₂ aqueous solution).

The two-fluid spray nozzle 30 converts a hydrogen peroxide solution into a hydrogen peroxide solution mist 31 by compressed air from a compressor (not shown) to supply the same to the inside of the isolator 10. In the present invention, the mist supply device is not restricted to a two-fluid spray nozzle, and the mist generation mechanism and output are not particularly restricted.

Herein, the mist circulation dispersion unit 40 will be described. In this first embodiment, the mist circulation dispersion unit 40 include 2 vibration boards 41, 42. The 2 vibration boards 41, 42 are disposed at 2 portions: a lower portion of a right wall surface and an upper portion of a left wall surface shown inside the isolator 10 against side wall surfaces 12, 13 such that vibration surfaces 41a, 42a face horizontally inside the isolator 10. These 2 vibration boards 41, 42 are arranged without board surfaces (vibration surfaces) thereof being opposite each other (the board surfaces to face each other in front) . The reason for arranging the 2 vibration boards 41, 42 without being opposite each other and the action of the hydrogen peroxide solution mist 31 will be described later.

Herein, the vibration board 41 will be described (also applied to the vibration board 42). FIG. 2 is a schematic perspective view showing a plurality of ultrasonic speakers arranged on a speaker base in a vibration board included in the decontamination device in FIG. 1. In FIG. 2, the vibration board 41 includes a base and a plurality of transmitters. In this first embodiment, the base used is a speaker base 45, and the transmitter used is an ultrasonic speaker 46. In this first embodiment, 25 ultrasonic speakers 46 are arranged on a plain surface 45a of the speaker base 45 so as to be uniform in transmission direction of a vibration surface 46a (leftward as seen from the front of the speaker base 45 shown). The number of ultrasonic speakers is not particularly restricted.

In this first embodiment, the ultrasonic speaker 46 used is an ultra-directional ultrasonic speaker. Specifically, an ultrasonic speaker (DC12V, 50mA) of frequency modulation system for transmitting an ultrasound whose frequency is around 40 KHz is used. The type, size, structure and output of the ultrasonic speaker are not particularly restricted. In the present invention, the vibration board included in the mist circulation dispersion unit is not restricted to an ultrasonic speaker, and the ultrasonic generation mechanism, frequency range and output are not particularly restricted.

In this first embodiment, a plurality of (25) ultrasonic speakers 46 are arranged so as to be uniform in transmission direction of the vibration surface 46a, and the transmitters are operated in the same phase to mutually amplify ultrasounds from the plurality of ultrasonic speakers 46 in the front direction and mutually cancel out ultrasounds from the plurality of ultrasonic speakers 46 in the lateral direction. Consequently, the ultrasonic speakers 46 arranged on the speaker base 45 are subjected to ultrasonic vibration to generate a significantly directional sound flow traveling in the air from each of the vibration surfaces 46a in the vertical direction. By controlling the frequency and output of the ultrasonic speakers 46 using a control unit (not shown), efficient decontamination operations can be performed.

Subsequently, the action of the hydrogen peroxide solution mist 31 inside the isolator 10 including the decontamination device 20 according to the above configuration will be described. In FIG. 1, the vibration board 41 placed at a right lower portion shown inside the isolator 10 allows a vibration surface 41a thereof to face in the left direction in the figure (in the same direction as the direction of the vibration surface 46a of the ultrasonic speaker 46).

Ultrasonic vibration of the ultrasonic speaker 46 in this state allows a significantly directional sound flow 41b traveling in the vertical direction (in the left direction shown) from the vibration surface 41a to take in the hydrogen peroxide solution mist 31 discharged from the two-fluid spray nozzle 30 to generate a pressing force by acoustic radiation pressure and move the hydrogen peroxide solution mist 31 in the direction of the sound flow 41b (in the left direction shown). In this case, the hydrogen peroxide solution mist 31 is converted into a fine mist 31a refined by the ultrasonic vibration from the sound flow 41b, and circulated and dispersed inside the isolator 10.

Meanwhile, the vibration board 42 placed at a left upper portion shown inside the isolator 10 allows the vibration surface 42a thereof to face in the right direction in the figure (in the same direction as the direction of the vibration surface 46a of the ultrasonic speaker 46). Ultrasonic vibration of the ultrasonic speaker 46 in this state allows a significantly directional sound flow 42b traveling in the vertical direction (in the right direction shown) from the vibration surface 42a to press the fine mist 31a refined and fed by the sound flow 41b from acoustic radiation pressure and move the same in the direction of the sound flow 42b (in the right direction shown). In this case, the fine mist 31a is converted into a more stable fine mist 31b by ultrasonic vibration from the sound flow 42b and circulated and dispersed inside the isolator 10.

Accordingly, the vibration boards 41 and 42 are disposed inside the isolator 10 such that the respective vibration surfaces 41a, 42a are not opposite each other in front. In cases where the vibration surface 41a of the vibration board 41 and the vibration surface 42a of the vibration board 42 are opposite each other in front, the vibration boards 41 and 42 generate ultrasounds, their interaction generates a stationary sound wave field. This is attributed to the inability of the fine mists 31a, 31b to move due to no pressing force by acoustic radiation pressure.

Thus, the fine mists 31a, 31b refined and stabilized by the sound flows 41b and 42b circulate so as to rotate in the arrow direction shown (clockwise) inside the isolator 10. The sound flows 41b and 42b, which are a stable stationary longitudinal wave traveling on a plain surface, are transmitted as airflow having no difference in wind velocity compared to a direct type from a mist nozzle or a fan type.

In fact, since the fine mists 31a, 31b are refined by ultrasonic vibration and have smaller particle sizes and larger surface areas, it is believed that the evaporation efficiency of mists is high, resulting in repeated evaporation and condensation. The fine mists 31a, 31b are highly-refined mists to form a uniform and thin condensed film on an internal wall surface of the isolator 10. Therefore, as opposed to conventional decontamination operations, no partial, uneven or thick condensed film is formed on the internal wall surface of the isolator 10.

Thus, the fine mists 31a, 31b of hydrogen peroxide are subjected to constant ultrasonic vibration to be circulated with repeated evaporation, condensation, and refinement inside the isolator 10. Even on the internal wall surface of the isolator 10, the fine mists 31a, 31b are subjected to constant ultrasonic vibration to cause repeated re-evaporation and condensation of a uniform and thin condensed film. Accordingly, it is believed that ultrafine particles of hydrogen peroxide 3µm or less and a hydrogen peroxide gas are subjected to phase change for coexistence inside the isolator 10 to provide a high-level decontamination environment.

Also, by repeated re-evaporation and condensation of the uniformly and thinly formed condensed film on the internal wall surface of the isolator 10, the concentration of a decontamination agent in a mist for decontamination can be increased and efficient decontamination can be performed with a small amount of decontamination agent. Such an efficient decontamination with a small amount of decontamination agent can improve the efficiency of aeration after decontamination and reduce the duration of decontamination operations. Furthermore, the secondary effect is that ultrasonic vibration and acoustic radiation pressure by the sound flows 41b and 42b can remove a deposit on the internal wall surface of the isolator 10.

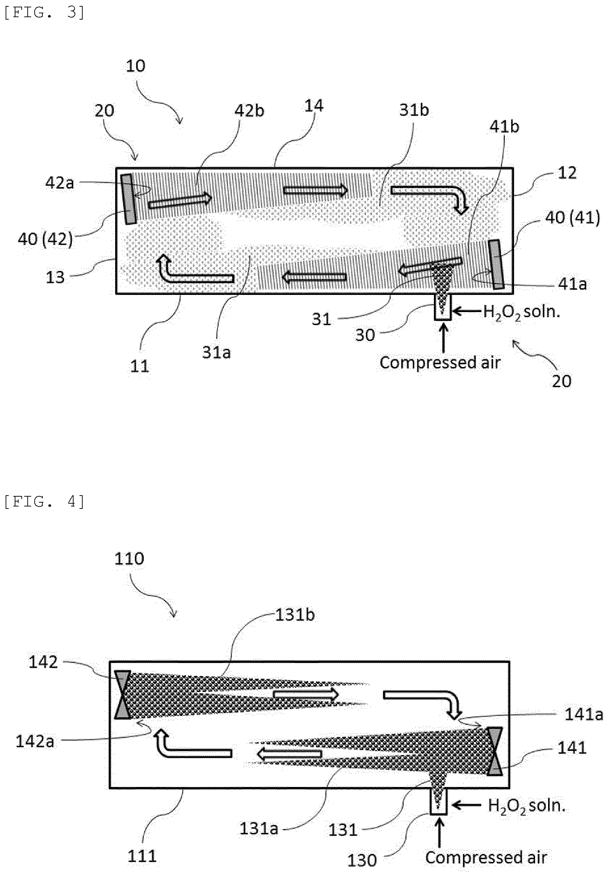

Herein, an alternative of this first embodiment will be described. As above described in in FIG. 1, the 2 vibration boards 41, 42 are disposed at the 2 portions: the lower portion of the right wall surface and the upper portion of the left wall surface shown inside the isolator 10 (parallel to the side wall surfaces) against the side wall surfaces 12, 13 such that the vibration surfaces 41a, 42a face horizontally inside the isolator 10. In the alternative of this first embodiment, the directions of the vibration boards 41, 42 are changeable. FIG. 3 is a schematic cross-sectional view showing the inside of an alternative of the isolator in FIG. 1 viewed from a side.

In the alternative in FIG. 3, 2 vibration boards 41, 42 are disposed at 2 portions: a lower portion of a right wall surface and an upper portion of a left wall surface shown inside an isolator 10 against side wall surfaces 12, 13 such that vibration surfaces 41a, 42a face in the direction of tilting the vibration surfaces 41a, 42a at predetermined angles with the side wall surfaces 12, 13. Specifically, the vibration board 41 disposed at the lower portion of the right wall surface shown is placed in the direction of tilting the vibration surface 41a thereof toward a bottom wall surface 11 from the side wall surface 12. Meanwhile, the vibration board 42 disposed at the upper portion of the left wall surface shown is placed in the direction of tilting the vibration surface 42a thereof toward an upper wall surface 14 from the side wall surface 13.

In FIG. 3 showing that the 2 vibration boards 41, 42 are disposed, the vibration board 41 placed at a right lower portion shown inside the isolator 10 is tilted by allowing the vibration surface 41a thereof to face the bottom wall surface 11 in the left lower direction shown. Ultrasonic vibration of the ultrasonic speaker 46 in this state allows a significantly directional sound flow 41b traveling in the vertical direction (in the left lower direction shown) from the vibration surface 41a to take in the hydrogen peroxide solution mist 31 discharged from the two-fluid spray nozzle 30 to generate a pressing force by acoustic radiation pressure and move the hydrogen peroxide solution mist 31 along the bottom wall surface 11 in the direction of the sound flow 41b (in the left lower direction shown). In this case, the hydrogen peroxide solution mist 31 is converted into a fine mist 31a refined by the ultrasonic vibration from the sound flow 41b, and circulated and dispersed inside the isolator 10, particularly adjacent to the bottom wall surface 11.

Meanwhile, the vibration board 42 placed at the left upper portion shown inside the isolator 10 is tilted by allowing the vibration surface 42a thereof to face the upper wall surface 14 in the right upper direction in the figure. Ultrasonic vibration of the ultrasonic speaker 46 in this state allows a significantly directional sound flow 42b traveling in the vertical direction (in the right upper direction shown) from the vibration surface 42a to press the fine mist 31a refined and fed by the sound flow 41b from acoustic radiation pressure and move the same along the upper wall surface 14 in the direction of the sound flow 42b (in the right direction shown) . In this case, the fine mist 31a is converted into a more stable fine mist 31b by ultrasonic vibration from the sound flow 42b and circulated and dispersed inside the isolator 10, particularly adjacent to the upper wall surface 14.

Accordingly, the vibration boards 41, 42 are disposed such that the respective vibration surfaces 41a, 42a are tilted to face the bottom wall surface 11 or the upper wall surface 14. Thus, the fine mists 31a, 31b refined and stabilized by the sound flows 41b and 42b circulate so as to rotate in the arrow direction shown (clockwise), and particularly along each of the wall surfaces inside the isolator 10. The resulting increased density of the mist adjacent to each of the wall surfaces is expected to further improve the decontamination efficiency.

Herein, conventional decontamination effects are compared to those in this first embodiment. FIG. 4 is a schematic cross-sectional view showing the inside of a conventional isolator viewed from a side. In FIG. 4, only a clean area inside the isolator is described as in FIG. 1, and an air supply fan, an air discharge fan, an HEPA filter, a control unit, and each of the devices placed inside are not described.

In FIG. 4, the isolator 110 includes a mist supply unit 130 for supplying a mist for decontamination to the inside of a bottom wall surface 111, and 2 circulating fans 141, 142. Herein, the mist supply unit used is a two-fluid spray nozzle 130, which is the same as in FIG. 1, and the decontamination agent used is a hydrogen peroxide solution (H₂O₂ aqueous solution). The two-fluid spray nozzle 130 converts a hydrogen peroxide solution into a hydrogen peroxide solution mist 131 by compressed air from a compressor (not shown) to supply the same to the inside of the isolator 110.

The 2 circulating fans 141, 142 are disposed at 2 portions: a lower portion of a right wall surface and an upper portion of a left wall surface shown inside the isolator 110 against side wall surfaces such that blowing surfaces 141a, 142a face horizontally inside the isolator 110. These 2 circulating fans 141, 142 are arranged without blowing surfaces 141a, 142a being opposite each other.

Subsequently, the action of the hydrogen peroxide solution mist 131 inside the isolator 110 according to the above configuration will be described. In FIG. 4, the circulating fan 141 disposed at a right lower portion shown inside the isolator 110 is disposed such that the blowing surface 141a thereof faces in the left direction shown.

The activation of the circulating fan 141 in this state allows a blowing air traveling in the vertical direction (in the left direction shown) from the blowing surface 141a to take in a hydrogen peroxide solution mist 131 discharged from the two-fluid spray nozzle 130 to move the same in the traveling direction (in the left direction shown). In this case, the hydrogen peroxide solution mist 131 is not refined, and moves inside the isolator 110 as a mist 131a while forming a coarse mist by mist integration.

Meanwhile, the circulating fan 142 placed at right upper portion shown inside the isolator 110 allows the blowing surface 142a thereof to face in the right direction shown. The activation of the circulating fan 142 in this state generates a blowing air traveling in the vertical direction (in the right direction shown) from the blowing surface 142a. In this case, the mist 131a fed from the circulating fan 141 is not refined and moves inside the isolator 110 as a mist 131b while forming a coarse mist by mist integration.

Thus, the mists 131a, 131b circulate so as to rotate in the arrow direction shown (clockwise) by the circulating fans 141, 142 inside the isolator 110. However, the blowing airs by the circulating fans 141, 142 are transmitted not as a stable stationary wave, but as airflow having a difference in wind velocity and variable wind velocity. The mists 131a, 131b are not refined and cause partial and uneven condensation on an internal wall surface of the isolator 110 while forming a coarse mist by mist integration.

In fact, since the mists 131a, 131b are not refined and have larger particle sizes and smaller area surfaces, the evaporation efficiency of mists is low, which unfortunately fails to achieve uniform humidification and uniform decontamination of the entire internal space inside the isolator 110. Thus, the concentration of a mist for decontamination cannot be increased, thereby making it difficult to reduce the amount of a decontamination agent. Also in conventional decontamination operations, the aeration efficiency after a decontamination work is low, and the reduction in the duration of such decontamination operations is problematic. Therefore, it is found that the use of the decontamination device according to this first embodiment is significant as opposed to conventional decontamination operations.

Subsequently, the action of decontamination of the isolator 10 including the decontamination device 20 according to this first embodiment will be described by reference to examples. The present invention is not restricted to the following examples.

### EXAMPLE

In this example, a simulated isolator used is a closed space (having an internal wall surface made of stainless steel) with a volume of 0.4 m³, and 2 vibration boards were placed as in this first embodiment (see FIG. 1). The mist supply unit for converting hydrogen peroxide (35 W/V%) into a mist used is an ultrasonic humidifier (nebulizer) in place of a two-fluid spray nozzle. The input of a hydrogen peroxide solution into the closed space was determined by an input speed of 0.4 g/min by 10 minutes (equal to 10 g/m³). In the closed space during decontamination, the temperature was 23 °C, and the humidity was 50 %. Thereafter, a 25-minute aeration removed the hydrogen peroxide in the closed space.

Decontamination effects in the closed space were confirmed by an enzyme indicator (EI). EI is an apparatus for fluorescence assay of residual enzymatic activity after a test to confirm decontamination effects, and this approach is advantageous in removing culture operations in conventional biological indicator (BI) and reducing the duration of operations. EI's comparative equality with BI was recently confirmed and the EI technique has proactively been used. The log spore reduction (LRD) value was calculated by the logarithmic decrement of fungi from the EI's fluorescence intensity after decontamination, and the LRD of 4 to 6 or more was judged as a sufficiently acceptable decontamination standard effect inside the isolator.

The positions of EIs in the closed space as a simulated isolator is shown in FIG. 1. In FIG. 1, EI-1 and EI-2 are placed on the near side of a left upper portion and the far side of the left upper portion in the figure, respectively. EI-3 and EI-4 are placed on the near side of a left lower portion and the far side of the left lower portion in the figure, respectively. EI-5 and EI-6 are placed on the near side of a right upper portion and the far side of the right upper portion in the figure, respectively. EI-7 and EI-8 are placed on the near side of a right lower portion and the far side of the right lower portion in the figure, respectively. The case where 2 vibration boards disposed in the closed space are operated is Example, and the case where the 2 vibration boards are not operated is Comparative Example. Table 1 shows the LRD values of EI-1 to EI-8 of Example and Comparative Example after each decontamination operation.

**Table 1**

| | EI1 | EI2 | EI3 | EI4 | EI5 | EI6 | EI7 | EI8 |
|---|---|---|---|---|---|---|---|---|
| Example | 5.9 | 6.7 | 7.6 | 7.4 | 7.9 | 8.4 | 6.4 | 7.7 |
| Comparative Example | 4.6 | <2.5 | <2.5 | 6.5 | <2.5 | <2.5 | 7.9 | 6.2 |

As shown in Table 1, the LDR values in the closed space by operating the 2 vibration boards are found to provide sufficient effects at any position, resulting in uniform decontamination. In contrast, the LRD values in Comparative Example show many insufficiently decontaminated areas, which fail to reach the value of 4. Despite no test confirmation, complete decontamination of a closed space having this volume by the circulating fan system (see FIG. 4) requires a hydrogen peroxide solution determined by the input velocity of 0.7 g/min by 25 minutes (equal to 44 g/m³) as an empirical value, and the duration of the following aeration is 45 minutes. Accordingly, the effect by the decontamination device according to this first embodiment is obviously to significantly reduce the input of a hydrogen peroxide solution. It is consequently found that the duration of aeration after decontamination is reduced.

Thus, according to this first embodiment, a decontamination device capable of accomplishing a decontamination effect with a proper amount of decontamination agent supplied to a room to be decontaminated by employing a mist circulation dispersion mechanism and reducing the duration of operations such as aeration to achieve more efficient decontamination works can be provided.

### <Second embodiment>

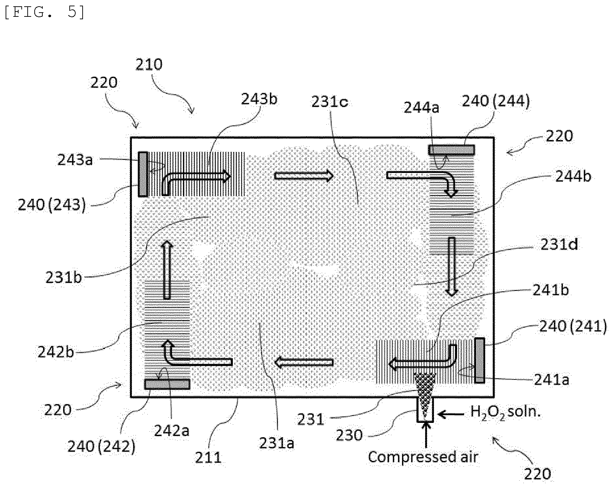

While the above first embodiment relates to a decontamination device including 2 vibration boards, this second embodiment relates to a decontamination device placed in an isolator including 4 vibration boards, which will be described. FIG. 5 is a schematic cross-sectional view showing the inside of the isolator including the decontamination device according to the second embodiment viewed from a side.

In FIG. 5, an isolator 210 includes a decontamination device 220 inside thereof. The decontamination device 220 is composed of a mist supply unit 230, a mist circulation dispersion unit 240, and a control unit (not shown). In this second embodiment, the mist supply unit 230 used is a two-fluid spray nozzle 230, which is the same as in the above first embodiment, and is placed on a bottom wall surface 211 of the isolator 210. In this second embodiment, the decontamination agent used is a hydrogen peroxide solution (H₂O₂ aqueous solution), which is the same as in the above first embodiment.

The two-fluid spray nozzle 230 converts a hydrogen peroxide solution into a hydrogen peroxide solution mist 231 by compressed air from a compressor (not shown) to supply the same to the inside of the isolator 210. In the present invention, the mist supply device is not restricted to a two-fluid spray nozzle, and the mist generation mechanism and output are not particularly restricted.

Herein, a mist circulation dispersion unit 240 will be described. In this second embodiment, the mist circulation dispersion unit 240 include 4 vibration boards 241, 242, 243, 244. The 4 vibration boards 241, 242, 243, 244 are disposed at 4 portions: a lower portion of a right wall surface, a lower portion of a left wall surface, an upper portion of a left wall surface, and an upper portion of a right wall surface, respectively, shown inside the isolator 210.

The vibration boards 241, 243 are disposed at the lower portion of the right wall surface and the upper portion of the left wall surface shown against the right and left side wall surfaces such that vibration surfaces 241a, 243a face horizontally inside the isolator 210. Meanwhile, the vibration boards 242, 244 are disposed at the lower portion of the left wall surface and the upper portion of the right wall surface shown against the bottom wall surface and upper wall surface such that vibration surfaces 242a, 244a face vertically inside the isolator 210. These 4 vibration boards 241, 242, 243, 244 are arranged without the boards surfaces (vibration surfaces) being opposite each other.

Herein, the vibration boards 241, 242, 243, 244 will be described. These vibration boards 241, 242, 243, 244 used are the same in structure as the vibration boards 41, 42 in the above first embodiment (see FIG. 2). The frequency and output of these vibration boards 241, 242, 243, 244 used are also the same as those of the vibration boards in the above first embodiment.

Subsequently, the action of the hydrogen peroxide solution mist 231 inside the isolator 210 including the decontamination device 220 according to the above configuration will be described. In FIG. 5, the vibration board 241 placed at the right lower portion shown inside the isolator 210 allows the vibration surface 241a thereof to face in the left direction shown.

Ultrasonic vibration of the ultrasonic speaker 46 in this state allows a significantly directional sound flow 241b traveling in the vertical direction (in the left direction shown) from the vibration surface 241a to take in the hydrogen peroxide solution mist 231 discharged from the two-fluid spray nozzle 230 to generate a pressing force by acoustic radiation pressure and move the hydrogen peroxide solution mist 231 in the direction of the sound flow 241b (in the left direction shown). In this case, the hydrogen peroxide solution mist 231 is converted into a fine mist 231a refined by the ultrasonic vibration from the sound flow 241b, and circulated and dispersed inside the isolator 210.

Subsequently, the vibration board 242 placed at a left lower portion shown inside the isolator 210 allows the vibration surface 242a thereof to face in the upper direction in the figure. Ultrasonic vibration of the ultrasonic speaker 46 in this state allows a significantly directional sound flow 242b traveling in the vertical direction (in the upper direction shown) from the vibration surface 242a to press the fine mist 231a refined and fed by the sound flow 241b from acoustic radiation pressure and move the same in the direction of the sound flow 242b (in the upper direction shown). In this case, the fine mist 231a is converted into a more stable fine mist 231b by ultrasonic vibration from the sound flow 242b and circulated and dispersed inside the isolator 210.

Subsequently, the vibration board 243 placed at the left upper portion shown inside the isolator 210 allows the vibration surface 243a thereof to face in the right direction in the figure. Ultrasonic vibration of the ultrasonic speaker 46 in this state allows a significantly directional sound flow 243b traveling in the vertical direction (in the right direction shown) from the vibration surface 243a to press the fine mist 231b refined and fed by the sound flow 242b from acoustic radiation pressure and move the same in the direction of the sound flow 243b (in the right direction shown). In this case, the fine mist 231b is converted into a more stable fine mist 231c by ultrasonic vibration from the sound flow 243b and circulated and dispersed inside the isolator 210.

Subsequently, the vibration board 244 placed at the right upper portion shown inside the isolator 210 allows the vibration surface 244a thereof to face in the lower direction shown. Ultrasonic vibration of the ultrasonic speaker 46 in this state allows a significantly directional sound flow 244b traveling in the vertical direction (in the lower direction shown) from the vibration surface 244a to press the fine mist 231c refined and fed by the sound flow 243b from acoustic radiation pressure and move the same in the direction of the sound flow 244b (in the lower direction shown). In this case, the fine mist 231c is converted into a more stable fine mist 231d by ultrasonic vibration from the sound flow 244b and circulated and dispersed inside the isolator 210.

Thus, the fine mists 231a, 231b, 231c, 231d refined and stabilized by the sound flows 241b, 242b, 243b, 244b circulate so as to rotate in the arrow direction shown (clockwise) inside the isolator 210. The sound flows 241b, 242b, 243b, 244b, which are a stable stationary longitudinal wave traveling on a plain surface, are transmitted as airflow having no difference in wind velocity compared to a direct type from a mist nozzle or a fan type. In this case, the fine mists 231a, 231b, 231c, 231d are highly refined mists, and generate no partial or uneven condensation on an internal wall surface of the isolator 210.

Also, since the fine mists 231a, 231b, 231c, 231d are refined by ultrasonic vibration and have smaller particle sizes and larger area surfaces, the evaporation efficiency of mists is high, resulting in uniform humidification and decontamination of the entire internal space of the isolator 210. Accordingly, the concentration of a mist for decontamination can be increased and efficient decontamination can be performed with a small amount of decontamination agent. Such an efficient decontamination with a small amount of decontamination agent can improve the efficiency of aeration after decontamination and reduce the duration of decontamination operations. Furthermore, the secondary effect is that ultrasonic vibration and acoustic radiation pressure by the sound flows 241b, 242b, 243b, 244b ca remove a deposit on the internal wall surface of the isolator 210.

Thus, according to this second embodiment, a decontamination device capable of accomplishing a decontamination effect with a proper amount of decontamination agent supplied to a room to be decontaminated by employing a mist circulation dispersion mechanism and reducing the duration of operations such as aeration to achieve more efficient decontamination works can be provided.

The present invention is achieved by not only each of the above embodiments, but also by the following various alternatives.
(1) In each of the above embodiments, an isolator is illustrated as a room to be decontaminated. However, the room to be decontaminated is not restricted thereto, and a clean room, a LABS, or a pass box may be a subject for decontamination. Also, a mist supply unit or a mist circulation dispersion unit (vibration board) of a decontamination device may be fixed in a working chamber, or may be introduce only for decontamination.
(2) In each of the above embodiments, the case where the number of vibration boards of a mist circulation dispersion unit is 2 and 4 is described. However, the number of vibration boards is not restricted thereto in the present invention. Therefore, a decontamination agent mist may be circulated in a room to be decontaminated using one vibration board. Also, 5 or more vibration boards may be placed at predetermined positions.
(3) In each of the above embodiments, a mist supply unit used is a two-fluid spray nozzle. However, the mist supply unit is not restricted thereto, and an ultrasonic humidifier (nebulizer) used in the example or a singlefluid spray nozzle may be used. Also, a combination of a plurality of mist supply units may be used.
(4) In each of the above embodiments, a vibration board of a mist circulation dispersion unit used includes a plurality of ultrasonic speakers placed on a speaker base. However, the vibration board is not restricted thereto, and any type of vibration board may be used so long as it includes a Langevin type transducer fixed to a stainless steel having a constant area or a board surface for ultrasonic vibration.
(5) In each of the above embodiments, a vibration board of a mist circulation dispersion unit used includes a plurality of ultrasonic speakers placed on a speaker base such that the ultrasonic speakers are uniform in transmission direction and these ultrasonic speakers are operated in the same phase. However, the vibration board of a mist circulation dispersion unit is not restricted thereto, and a plurality of ultrasonic speakers may be operated in a different phase.
(6) In each of the above embodiments, a decontamination agent used is a hydrogen peroxide solution (H₂O₂ aqueous solution). However, the decontamination agent is not restricted thereto, and it may be any type of decontamination agent so long as it is liquid.
(7) In each of the above embodiments, the circulating direction of a decontamination agent mist is vertical direction. However, the circulating direction is not restricted thereto, and it may be horizontal direction.

### REFERENCE SIGNS LIST

10, 110, 210...Isolator, 11, 111, 211...Bottom wall surface, 12, 13...Side wall surface, 14...Upper wall surface, 20, 220...Decontamination device, 30, 130, 230...Mist supply means (two-fluid spray nozzle), 31, 131, 231...Hydrogen peroxide solution mist, 131a, 131b...Mist, 31A, 31b, 231a, 231b, 231c, 231d...Mist, 40. 240...Mist circulation dispersion mechanism, 41, 42, 241, 242, 243, 244...Vibration board, 41A, 42a, 241a, 242a, 243a, 244a...Vibration surface, 41b, 42b, 241b, 242b, 243b, 244b...Sound flow, 45...Speaker base, 45a...Plain surface of speaker base, 46...Ultrasonic speaker, 46a...Vibration surface of ultrasonic speaker, 141, 142...Circulating fan, 141a, 142a...Blowing surface, EI-1 to EI-8...Enzyme indicator.

## Claims

1. A decontamination device placed inside a working chamber,
the decontamination device comprising a mist supply means and a mist circulation dispersion mechanism, wherein
the mist supply means converts a chemical for decontamination into a mist for decontamination, and supplies the same to the inside of the working chamber,
the mist circulation dispersion mechanism includes vibration boards disposed adjacent to internal wall surfaces of the working chamber, and the vibration boards are subjected to ultrasonic vibration to generate sound flows from board surfaces by an ultrasound in the vertical direction, and
the mist for decontamination supplied to the inside of the working chamber is pressed by acoustic radiation pressure to circulate and disperse the mist for decontamination in the working chamber.

2. The decontamination device according to claim 1, wherein
the mist circulation dispersion mechanism comprises a plurality of vibration boards,
the plurality of vibration boards is arranged without board surfaces thereof being opposite each other to generate no stationary wave sound field by an ultrasound generated from each of the vibration boards, and consequently
the mist for decontamination moves so as to rotate inside the working chamber.

3. The decontamination device according to claim 1 or 2, wherein
the vibration board includes a base and a plurality of transmitters,
the plurality of transmitters is arranged on a plain surface of the base so as to be uniform in transmission direction, and the transmitters are operated in the same phase, and
a sound flow is generated by a significantly directional ultrasound from the board surface of the vibration board in the vertical direction by mutually amplifying ultrasounds from the plurality of transmitters in the front direction and mutually canceling out ultrasounds from the plurality of transmitters in the lateral direction.

4. The decontamination device according to any one of claims 1 to 3, wherein
the mist for decontamination supplied to the inside of the working chamber is further refined by ultrasonic vibration generated from the vibration board.

5. The decontamination device according to any one of claims 1 to 4, wherein
the decontamination device comprises a control means for changing the frequency and output of the ultrasound generated from the vibration board and/or for transmitting an ultrasound intermittently to control the moving speed of the mist for decontamination circulated and dispersed in the working chamber.
